(19) Europäisches Patentamt
European Patent Office
Office européen des brevets

(11) EP 0 876 339 B1

# (12) FASCICULE DE BREVET EUROPEEN

(45) Date de publication et mention de la délivrance du brevet:
**13.09.2000 Bulletin 2000/37**

(21) Numéro de dépôt: **96941731.0**

(22) Date de dépôt: **10.12.1996**

(51) Int Cl.$^7$: **C07C 319/04**, C07C 323/52

(86) Numéro de dépôt international:
**PCT/FR96/01970**

(87) Numéro de publication internationale:
**WO 97/21672 (19.06.1997 Gazette 1997/26)**

(54) **PROCEDE DE FABRICATION D'ACIDES MERCAPTOCARBOXYLIQUES A PARTIR D'ACIDES CARBOXYLIQUES INSATURES**

VERFAHREN ZUR HERSTELLUNG VON MERCATOCARBONSAEUREN AUS UNGESAETTIGTEN CARBONSAEUREN

METHOD FOR PREPARING MERCAPTOCARBOXYLIC ACIDS FROM UNSATURATED CARBOXYLIC ACIDS

(84) Etats contractants désignés:
**DE FR GB IT**

(30) Priorité: **11.12.1995 FR 9514638**

(43) Date de publication de la demande:
**11.11.1998 Bulletin 1998/46**

(73) Titulaire: **Atofina**
**92800 Puteaux (FR)**

(72) Inventeur: **LABAT, Yves**
**F-33110 Le Bouscat (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**FR-A- 1 485 266**
**GB-A- 670 702**
**GB-A- 1 358 019**

- **COLLECTION OF CZECHOSLOVAK CHEMICAL COMMUNICATIONS, vol. 21, no. 6, 1956, PRAGUE CS, pages 1651-1653, XP002010494 A. EMR, ET AL.: "Synthese der Thioäpfelsäure" cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 876 339 B1

## Description

La présente invention porte sur un nouveau procédé de fabrication des acides mercaptocarboxyliques représentés par la formule (I) :

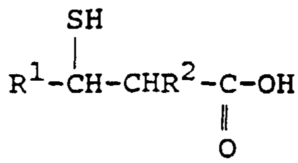

(I)

dans laquelle :

- $R^1$ représente hydrogène ou

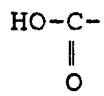
;

et

- $R^2$ représente hydrogène ou méthyle.

Ces acides mercaptocarboxyliques sont utiles comme intermédiaires de synthèse vers les esters (par exemple avec le 2-éthylhexanol, le pentaérythritol) utilisés soit pour obtenir les sels d'étain (stabilisants du PVC), soit comme agents durcisseurs ou modificateurs de résines époxydes (adhésifs), de polyuréthannes (verres optiques). L'acide mercaptopropionique peut aussi servir à modifier les polymères acryliques (industrie du papier, peinture).

On connaît différentes voies d'obtention d'acides mercaptocarboxyliques :

(1) L'une de ces voies consiste à faire réagir des acides carboxyliques saturés halogénés avec les sulfhydrates alcalins et sous pression de $H_2S$. Cette voie est abondamment décrite dans la littérature pour l'acide thioglycolique à partir de l'acide monochloracétique, et pour l'acide mercaptopropionique, à partir de l'acide chloro-3 propionique (voir brevet américain US-A-3 927 085). On sait que cette réaction est très sélective si les conditions opératoires sont bien choisies. Cependant, la stoechiométrie de la réaction nécessite l'utilisation de 2 moles de sulfhydrate alcalin par mole d'acide carboxylique chloré et la formation en conséquence de 2 moles de sel minéral à éliminer dans les effluents aqueux.

(2) Ont également été proposées des réactions d'ouverture de cycles lactoniques par les sulfhydrates alcalins ; ainsi, dans le brevet britannique n° 639 679, est décrite la formation de l'acide β-mercaptopropionique à partir de la β-propiolactone et de NaSH. On doit indiquer ici que la propiolactone n'est pas commercialement disponible.

(3) L'obtention de l'acide mercapto-3 propionique peut encore se réaliser à partir des matières premières largement disponibles que sont les acides organiques insaturés, et, en particulier, les dérivés acryliques.

. La réaction de $H_2S$ en milieu basique ou de sulfhydrates alcalins permet intermédiairement d'obtenir, en partant d'acrylonitrile, le mercaptopropionitrile, ultérieurement hydrolysable en acide mercapto-3 propionique.

Cependant, des conditions toutes particulières doivent être respectées pour éviter la réaction très aisée du mercaptopropionitrile formé avec l'acrylonitrile n'ayant pas réagi. Le sulfure thiodipropionitrile est, en effet, de formation très aisée, et il est alors nécessaire de procéder ultérieurement à une sulfhydrolyse du sulfure en mercaptan, ce qui complique le procédé d'obtention.

Par ailleurs, outre le fait de passer par des produits toxiques et instables (mercaptopropionitrile), ce procédé présente l'inconvénient que vont encore se former 2 moles de sel par mole d'acide mercapto-3 propionique.

. Une autre possibilité est celle consistant à utiliser, comme matière première, l'acrylate de méthyle, et à former, par réaction avec $H_2S$, le mercaptopropionate de méthyle, lequel, par hydrolyse, conduira à l'acide mercapto-3 propionique. Cette voie est économiquement peu attrayante, car elle s'effectue en deux étapes, avec perte d'une mole de méthanol.

. Une voie directe d'obtention de l'acide mercaptopropionique consiste à partir de l'acide acrylique, matière première bon marché. La réaction de l'acide acrylique en phase liquide, avec un large excès de $H_2s$ en présence de base organique comme catalyseur, est de mise en oeuvre peu aisée (voir FR-A-1 485 266). L'autre technique, consistant à faire réagir l'acide acrylique avec $CS_3Na_2$ permet d'améliorer la sélectivité en acide mercaptopropionique et d'opérer sans pression de $H_2S$. Malheureusement, l'utilisation de $CS_2$ et du méthanol comme co-solvant alourdit le procédé, surtout au regard du traitement des effluents.

GB-A-670 702 décrit, en son exemple I, la réaction suivante :

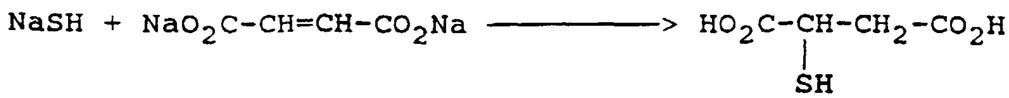

En fait, on prépare d'abord l'acide thiodisuccinique

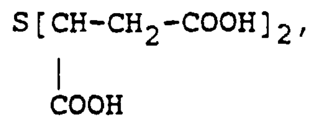

lequel subit une sulfhydrolyse en mercaptan du fait du pH basique. Autrement dit, d'une manière générale, on a d'abord formation d'un sulfure, lequel se sulfhydrolyse en mercaptan selon le schéma réactionnel :

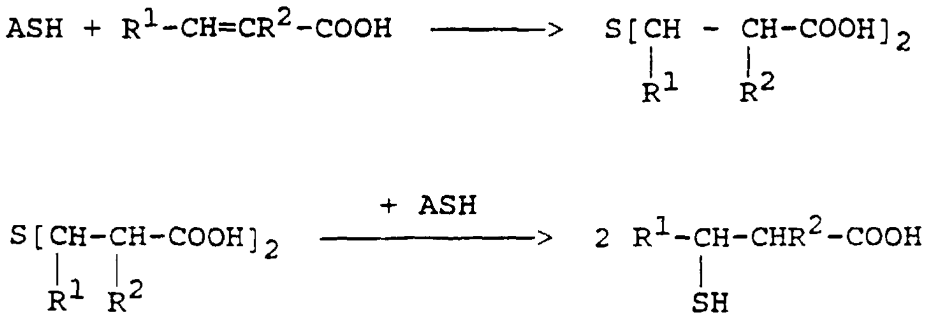


A, $R^1$, $R^2$ étant tels que définis ci-après.

La Société déposante a d'ailleurs confirmé expérimentalement ce schéma réactionnel. L'article de Collection of Czechoslovak Chemical Communications, Vol 21, n°6, 1956, Prague CS, pages 1651-1653 reprend ce qui est décrit dans GB-A-670 702.

GB-A-1 358 019 décrit un procédé de préparation de l'acide β-mercapto propionique par réaction de l'acide acrylique avec MSH où M = groupe ammonium ou métal alcalin ou alcalino-terreux en présence de disulfure de carbone $CS_2$. $CS_2$ joue le rôle de coréactif et empêche le sulfure de se former :

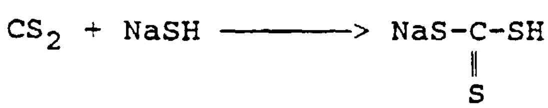

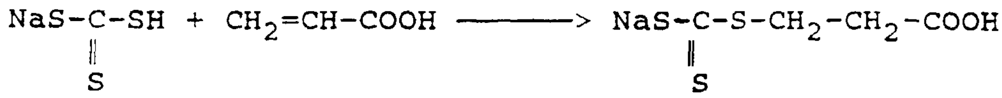

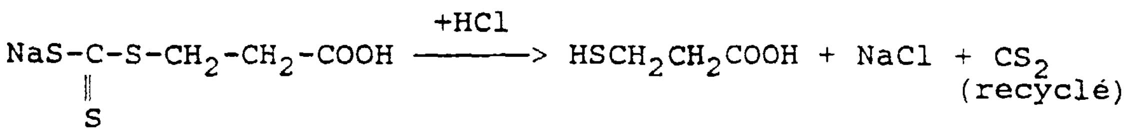


Conformément aux documents qui viennent d'être cités, on vise à obtenir une meilleure sélectivité en acides mercapto carboxyliques et à éviter la formation de sulfures. En fait, il s'agirait d'éviter la formation de sulfures par réaction du mercaptan déjà formé et du composé acrylique non transformé :

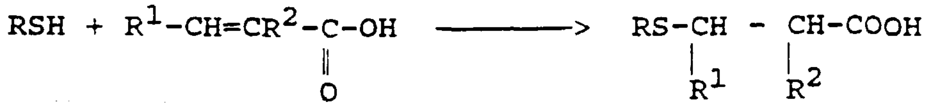

avec

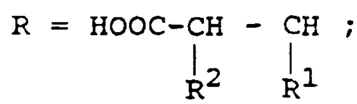

$R^1$ = H ou HOOC- et $R^2$ = H ou $CH_3$.

La présente invention est axée essentiellement sur l'effet bénéfique de la solubilité de $H_2s$ dans le milieu réactionnel qui favorise la formation de mercaptan. Conformément à la présente invention, le fait d'opérer avec un apport de $H_2S$ fait passer directement au mercaptan recherché, la sulfhydrolyse du sulfure

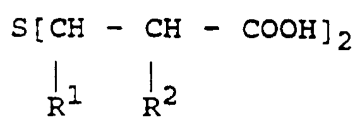

devenant impossible dans ces conditions.

Selon l'invention, on fait donc réagir des sulfhydrates avec les acides carboxyliques insaturés, selon la réaction globale suivante (cas où le sulfhydrate est représenté par ASH :

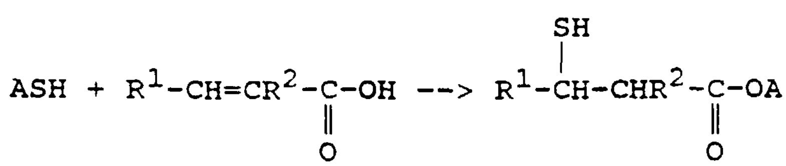

A = cation de métal alcalin ou ammonium ;
$R^1$ = H ou

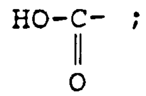

et
$R^2$ = H ou $CH_3$ qui est, en fait, la somme des deux réactions suivantes :

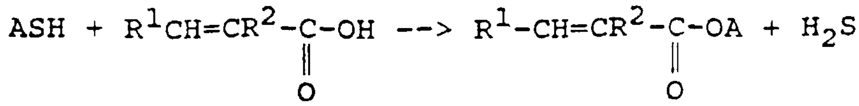

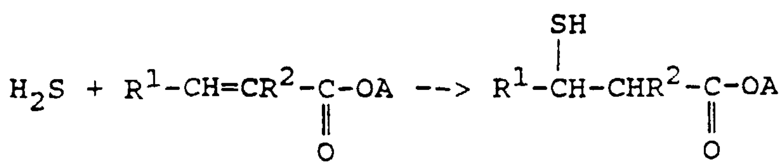

Il a été surprenant de constater que, dans les conditions précitées, le mercaptan formé réagi faiblement avec l'insaturation de l'acide $R^1$-CH=$CR^2$-COOH, alors que la réaction de formation du mercaptan est elle-même fortement favorisée. Ceci se traduit par de très bonnes sélectivités en le composé recherché de formule (I), supérieures à celles des procédés connus.

La présente invention a donc pour objet un procédé de fabrication d'un acide mercaptocarboxylique représenté par la formule (I) telle que définie auparavant, suivant lequel on fait réagir un acide carboxylique insaturé de formule (II) :

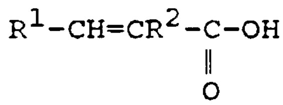 (II)

dans laquelle $R^1$ et $R^2$ sont tels que définis ci-dessus, avec un sulfhydrate de formule ASH, A étant un cation de métal alcalin ou un cation $NR^3R^4R^5R^{6+}$, $R^3$ à $R^6$ représentant chacun H ou radical hydrocarboné, ou de formule $Q(SH)_2$, Q étant un cation de métal alcalino-terreux,
et on acidifie le milieu réactionnel résultant pour obtenir le composé (I) recherché, caractérisé par le fait qu'on conduit la réaction en milieu aqueux, en milieu hydroalcoolique ou en milieu alcoolique avec un apport de $H_2S$ autre que celui apporté par la neutralisation de l'acide (II).

L'acide (II) de départ est choisi notamment parmi l'acide acrylique, l'acide méthacrylique, l'acide maléique et l'acide fumarique.

De manière générale, on peut effectuer la réaction selon l'invention en milieu aqueux, mais on peut obtenir une amélioration de la sélectivité en utilisant un milieu hydroalcoolique ou purement alcoolique. Ce milieu alcoolique, ou la fraction alcool du milieu hydroalcoolique, est formé par exemple par du méthanol, du propanol, de l'isopropanol ou un mélange de ces alcools.

Un paramètre important qui règle la sélectivité en acide mercapto carboxylique (I) est le rapport molaire $H_2S$/acide (II) en solution. Plus la concentration de $H_2S$ en solution sera grande et meilleure sera la sélectivité en acide (I). Si l'on opère sans apport de $H_2s$ autre que celui apporté par la neutralisation de l'acide (II), ce rapport est de 1. Si du $H_2S$ est apporté dans le système en réaction, sa solubilité dans le milieu réactionnel dépend de plusieurs facteurs physiques :

- dilution du milieu (le rapport molaire réel en solution $H_2S$/acide (II) sera plus élevé) ;
- utilisation d'un milieu alcoolique ou hydroalcoolique;
- augmentation de la pression en $H_2S$ ; et

- à un moindre degré, diminution de la température ; ainsi que du rapport $H_2S$/acide (II) en solution.

Pour augmenter ce rapport, comme cela est envisagé par la présente invention, on peut introduire $H_2S$ dans le milieu par apport extérieur direct et/ou produire $H_2S$ in situ par réaction du sulfhydrate avec au moins un acide introduit dans le milieu, conformément à la réaction suivante (avec ASH):

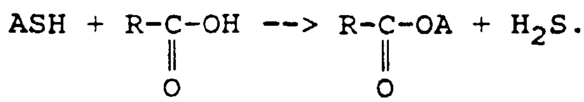

Cet acide peut être choisi parmi les acides organiques saturés de formule RCOOH, R représentant notamment un reste alkyle en $C_1$-$C_{18}$ pouvant porter des substituants tels qu'halogènes, et parmi les acides minéraux.

L'acide RCOOH est avantageusement choisi parmi l'acide acétique, l'acide propionique et l'acide chloro-3 propionique. Dans le cas où l'on souhaite obtenir de l'acide mercapto-3 propionique, on opère avantageusement en présence de l'acide chloro-3 propionique, lequel va lui-même conduire à l'acide mercapto-3 propionique recherché, conformément aux réactions suivantes :

$$2ASH + Cl\text{-}CH_2CH_2\text{-}COOH \rightarrow \underline{HS\text{-}CH_2\text{-}CH_2\text{-}COOA} + H_2S + ACl$$

$$ASH + CH_2{=}CH\text{-}COOH \rightarrow \underline{HS\text{-}CH_2\text{-}CH_2\text{-}COOA}$$

On peut également utiliser au moins un acide minéral, tel que HCl, $H_2SO_4$, etc. en lieu et place de l'acide organique.

Le pH doit avantageusement être de 6,5 à 8, et de préférence de 6,8 à 7,5 dans le milieu réactionnel sous pression de $H_2S$ pour éviter l'attaque nucléophile des espèces $^-S\text{-}Y\text{-}CO_2^-$ (Y = $CHR^1$-$CHR^2$) sur la double liaison pour conduire au sulfure.

Conformément à une autre caractéristique du procédé selon l'invention, on opère avantageusement sous une pression de $H_2S$ d'au moins 8 bars, notamment de 8 à 30 bars. Quant à la température de réaction, elle est avantageusement comprise entre 10 et 150°C. Dans le cas de l'obtention de l'acide mercapto-3 propionique, cette température est généralement de 20 à 100'C.

La concentration de l'acide (II), exprimée en moles par litre de solvant (eau ou alcool ou eau + alcool), est généralement comprise entre 0,5 et 4.

Le sulfhydrate ASH ou $Q(SH)_2$ peut être préparé à part et injecté dans le réacteur, ou bien être formé in situ, au moins en partie, par réaction en milieu aqueux ou alcoolique ou hydroalcoolique de $H_2S$ avec AOH ou $Q(OH)_2$, A et Q étant tels que définis ci-dessus. Ainsi, le sulfhydrate peut être obtenu par réaction de $H_2S$ avec la soude, la potasse, la chaux, les amines, l'ammoniaque.

Un des intérêts du procédé conforme à la présente invention est qu'il peut être appliqué dans des installations industrielles existantes, déjà optimisées pour la synthèse d'acides mercaptocarboxyliques à partir des acides carboxyliques chlorés (acide thioglycolique par exemple).

Il faut également souligner que le procédé de l'invention s'effectue en milieu aqueux ou hydroalcoolique ou alcoolique, sans utilisation de $CS_2$ et sans opérer en large excès de $H_2S$. Un autre intérêt économique est celui de ne former qu'une molécule de sel par molécule d'acide mercaptocarboxylique si on utilise $H_2S$ provenant d'une source extérieure, contrairement aux procédés partant d'acides carboxyliques halogénés, lesquels, lors de leur transformation en mercaptans, forment 2 moles de sel à éliminer.

Les exemples suivants illustrent encore la présente invention, sans toutefois en limiter la portée.

Exemple 1 : Préparation d'acide mercapto-3 propionique

Dans un autoclave de 2 litres et thermostaté, on introduit 440 g d'une solution aqueuse contenant 12,5 g de $NH_3$, soit 0,73 mole.

On introduit $H_2S$ dans l'autoclave pour maintenir la pression constante à 25 bars pendant la durée de l'essai. On porte la température à 40°C, et on introduit dans le réacteur à l'aide d'une pompe, 48 g d'acide acrylique, soit 0,66 mole.

L'essai est poursuivi une heure supplémentaire à 40°C sous 25 bars de $H_2S$. On refroidit alors le réacteur,

on chasse $H_2S$ excédentaire, on acidifie le milieu, on extrait la solution aqueuse par l'éther isopropylique, on évapore ce solvant, et on obtient l'acide mercapto-3 propionique brut (0,528 mole), soit 62 g d'un produit à 90% de pureté, lequel est ensuite distillé sous vide. Rendement : 80%.

Exemples 2a, 2b et 3 à 7

On procède comme à l'Exemple 1, excepté que l'on fait varier la concentration en acide acrylique et/ou la pression d'$H_2S$ ou qu'on introduit ou non de l'acide chloro-3 propionique ou qu'on opère en milieu méthanolique, la température moyenne étant de 40°C (plage de température = 20-60°C).

Les résultats sont rapportés dans le Tableau suivant.

Tableau

| Exemple | Nombre de moles d'acide acrylique/litre de solvant (eau ou méthanol) | Rapport molaire acide acrylique/acide chloro-3 propionique | Pression $H_2S$ (bar) | Milieu | Rendement en acide mercapto-3 propionique |
|---|---|---|---|---|---|
| 1 | 1,5 | sans acide chloro-3 propionique | 25 (1) | aqueux | 80 |
| 2a | 1 | sans acide chloro-3 propionique | 24 (1) | aqueux | 83 |
| 2b | | | 23 (2) | aqueux | 82 |
| 3 | 2 | sans acide chloro-3 propionique | 20 (1) | aqueux | 71 |
| 4 | 2,5 | sans acide chloro-3 propionique | 25 (1) | méthanolique | 87,5 |
| 5 | 2,5 | 1 | 18 | aqueux | 82 |
| 6 | 2 | 2 | 20 | aqueux | 81 |

(1) La pression de $H_2S$ est en partie réglée grâce à un apport extérieur de $H_2S$

(2) La pression de $H_2S$ est obtenue uniquement par la réaction des acides organiques et du $NH_4SH$. On opère ici avec un rapport molaire acide acrylique/acide acétique de 1.

Exemple 8 : Préparation de l'acide thiomalique

Dans un autoclave de 2 litres, on introduit 12 moles de soude dans environ 1900 g d'eau. On sature le milieu par de l'$H_2S$ et on maintient la pression à 10 bar. L'acide maléique préalablement dissous dans de l'eau, à raison de 2 moles (232 g) dans 365 g d'eau, est introduit dans l'autoclave par une pompe. On porte la température à 120°C que l'on maintient pendant 4 heures sous agitation.

Après refroidissement du réacteur, acidification et stripping, on obtient l'acide thiomalique en solution, à raison de 1,50 mole, soit 225 g. Ceci correspond à un rendement de 75% en acide thiomalique par rapport à l'acide maléique.

## Revendications

1. Procédé de fabrication d'un acide mercaptocarboxylique de formule (I) :

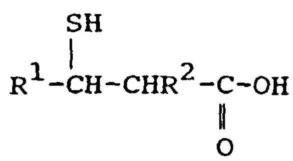

(I)

dans laquelle :

- $R^1$ représente hydrogène ou

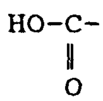 ;

et

- $R^2$ représente hydrogène ou méthyle,

suivant lequel on fait réagir un acide carboxylique insaturé de formule (II) :

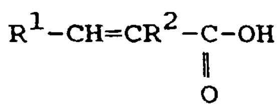

(II)

dans laquelle $R^1$ et $R^2$ sont tels que définis ci-dessus, avec un sulfhydrate de formule ASH, A étant un cation de métal alcalin ou un cation $NR^3R^4R^5R^{6+}$, $R^3$ à $R^6$ représentant chacun H ou radical hydrocarboné, ou de formule $Q(SH)_2$, Q étant un cation de métal alcalino-terreux ; et
on acidifie le milieu réactionnel résultant pour obtenir le composé (I) recherché, caractérisé par le fait qu'on conduit la réaction en milieu aqueux, en milieu hydroalcoolique ou en milieu alcoolique, avec un apport de $H_2S$ autre que celui apporté par la neutralisation de l'acide (II).

2. Procédé selon la revendication 1, caractérisé par le fait que l'on introduit $H_2S$ dans le milieu par apport extérieur direct.

3. Procédé selon la revendication 1, caractérisé par le fait que l'on produit $H_2S$ in situ par réaction du sulfhydrate avec au moins un acide introduit dans le milieu.

4. Procédé selon la revendication 3, caractérisé par le fait que l'acide introduit dans le milieu est choisi parmi les acides organiques saturés de formule RCOOH, R représentant notamment un reste alkyle en $C_1$-$C_{18}$ pouvant

porter des substituants tels qu'halogènes, et parmi les acides minéraux.

**5.** Procédé selon la revendication 4, caractérisé par le fait que l'acide RCOOH est choisi parmi l'acide acétique, l'acide propionique et l'acide chloro-3 propionique, l'acide RCOOH étant avantageusement l'acide chloro-3 propionique lorsque le procédé est destiné à la production de l'acide mercapto-3 propionique.

**6.** Procédé selon l'une des revendications 1 à 5, caractérisé par le fait que l'on opère sous une pression de $H_2S$ d'au moins 0,8 MPa (8 bars), notamment de 0,8 à 3 MPa (8 à 30 bars).

**7.** Procédé selon l'une des revendications 1 à 6, caractérisé en ce que l'on effectue la réaction à une température comprise entre 10 et 150°C.

**8.** Procédé selon l'une des revendications 1 à 7, caractérisé par le fait que le milieu alcoolique ou la fraction alcool du milieu hydroalcoolique est formé par du méthanol, du propanol, de l'isopropanol, ou par un mélange de ces alcools.

**9.** Procédé selon l'une des revendications 1 à 8, caractérisé en ce que l'acide (II) de départ est choisi parmi l'acide acrylique, l'acide méthacrylique, l'acide maléique et l'acide fumarique.

**10.** Procédé selon la revendication 7, conduisant à l'acide mercapto-3 propionique, caractérisé en ce que l'on effectue la réaction à une température de 20 à 100°C.

**11.** Procédé selon l'une des revendications 1 à 10, caractérisé par le fait que la concentration de l'acide (II), exprimée en moles par litre de solvant (eau ou alcool ou eau + alcool), est comprise entre 0,5 et 4.

**Patentansprüche**

**1.** Verfahren zur Herstellung einer Mercaptocarbonsäure der Formel (I)

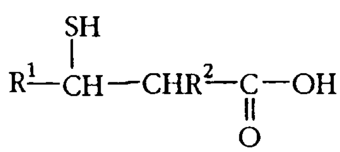

(I)

in der:

- $R^1$ Wasserstoff oder

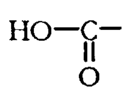

darstellt; und

- $R^2$ Wasserstoff oder Methyl darstellt,

wobei man eine ungesättigte Carbonsäure der Formel (II)

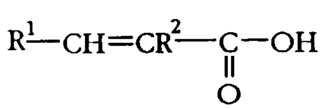

(II)

in der $R^1$ und $R^2$ wie zuvor definiert sind,
mit einem Sulfhydrat (Hydrogensulfid) der Formel ASH, in der A ein Alkalimetallkation oder ein Kation $NR^3R^4R^5R^{6+}$ ist, wobei $R^3$ bis $R^6$ jeweils H oder einen Kohlenwasserstoffrest darstellen, oder mit einem Sulfhydrat (Hydrogensulfid) der Formel $Q(SH)_2$, in der Q ein Erdalkalikation ist, reagieren läßt; und
man das resultierende Reaktionsmilieu ansäuert, um die gewünschte Verbindung (I) zu erhalten,
dadurch gekennzeichnet, daß man die Reaktion in wäßrigem Milieu, wäßrigalkoholischem Milieu oder alkoholischem Milieu mit einer weiteren $H_2S$-Zufuhr als derjenigen Zufuhr, die durch die Neutralisation der Säure (II) bereitgestellt wird, durchführt.

**2.** Verfahren nach Anspruch 1, dadurch gekennzeichnet, daß man $H_2S$ durch direkte äußere Zufuhr in das Milieu einbringt.

**3.** Verfahren nach Anspruch 1, dadurch gekennzeichnet, daß man $H_2S$ in situ durch Reaktion des Sulfhydrats (Hydrogensulfid) mit mindestens einer Säure erzeugt, die zu dem Reaktionsmilieu hinzugegeben wird.

**4.** Verfahren nach Anspruch 3, dadurch gekennzeichnet, daß die zu dem Milieu hinzugegebene Säure aus gesättigten organischen Säuren der Formel RCOOH, wobei R insbesondere einen $C_1$-$C_{18}$-Alkylrest darstellt, der Substituenten wie Halogene enthalten kann, und aus anorganischen Säuren, ausgewählt ist.

**5.** Verfahren nach Anspruch 4, dadurch gekennzeichnet, daß die Säure RCOOH ausgewählt ist aus Essigsäure, Propionsäure und 3-Chlorpropionsäure, wobei die Säure RCOOH vorzugsweise 3-Chlorpropionsäure ist, wenn das Verfahren zur Herstellung von 3-Mercaptopropionsäure bestimmt ist.

**6.** Verfahren nach einem der Ansprüche 1 bis 5, dadurch gekennzeichnet, daß man unter einem $H_2S$-Druck von mindestens 0,8 MPa (8 bar), insbesondere von 0,8 bis 3 MPa (8 bis 30 bar), verfährt.

**7.** Verfahren nach einem der Ansprüche 1 bis 6, dadurch gekennzeichnet, daß man die Reaktion bei einer Temperatur zwischen 10 und 150 °C durchführt.

**8.** Verfahren nach einem der Ansprüche 1 bis 7, dadurch gekennzeichnet, daß das alkoholische Milieu oder die Alkoholfraktion des wäßrig-alkoholischen Milieus durch Methanol, Propanol, Isopropanol oder durch eine Mischung dieser Alkohole gebildet wird.

**9.** Verfahren nach einem der Ansprüche 1 bis 8, dadurch gekennzeichnet, daß die Ausgangssäure (II) ausgewählt ist aus Acrylsäure, Methacrylsäure, Maleinsäure und Fumarsäure.

**10.** Verfahren nach Anspruch 7, das zur 3-Mercaptopropionsäure führt, dadurch gekennzeichnet, daß man die Reaktion bei einer Temperatur von 20 bis 100 °C durchführt.

**11.** Verfahren nach einem der Ansprüche 1 bis 10, dadurch gekennzeichnet, daß die Konzentration der Säure (II), berechnet in Mol pro Liter Lösungsmittel (Wasser oder Alkohol oder Wasser + Alkohol), zwischen 0,5 und 4 liegt.

**Claims**

**1.** Process for the manufacture of a mercaptocarboxylic acid of formula (I) :

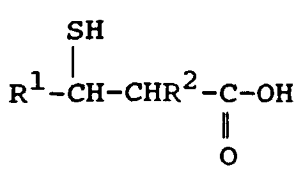

(I)

in which :

- $R^1$ represents hydrogen or

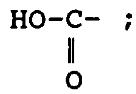

and

- $R^2$ represents hydrogen or methyl,

according to which an unsaturated carboxylic acid of formula (II) :

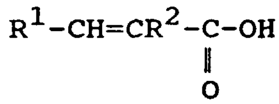 (II)

in which $R^1$ and $R^2$ are as defined above, is reacted with a hydrosulphide of formula ASH, A being an alkali metal cation or a cation $NR^3R^4R^5R^{6+}$, $R^3$ to $R^6$ each representing H or a hydrocarbon radical, or of formula $Q(SH)_2$, Q being an alkaline-earth metal cation ; and
the resulting reaction medium is acidified in order to obtain the desired compound (I), characterized in that the reaction is carried out in an aqueous medium, an aqueous-alcoholic medium or an alcoholic medium, with a supply of $H_2S$ other than that supplied by the neutralization of the acid (II).

**2.** Process according to Claim 1, characterized in that $H_2S$ is introduced into the medium by a direct external supply.

**3.** Process according to Claim 1, characterized in that $H_2S$ is produced in situ by reacting the hydrosulphide with at least one acid introduced into the medium.

**4.** Process according to Claim 3, characterized in that the acid introduced into the medium is chosen from the saturated organic acids of formula RCOOH, R representing especially a $C_1$-$C_{18}$ alkyl residue capable of carrying substituents such halogens, and from inorganic acids.

**5.** Process according to Claim 4, characterized in that the acid RCOOH is chosen from acetic acid, propionic acid, 3-chloropropionic acid, the acid RCOOH being advantageously 3-chloropropionic acid when the process is intended for the production of 3-mercaptopropionic acid.

**6.** Process according to one of Claims 1 to 5, characterized in that the procedure is carried out under an $H_2S$ pressure of at least 8 bar, especially from 8 to 30 bar.

**7.** Process according to one of Claims 1 to 6, characterized in that the reaction is carried out at a temperature of between 10 and 150°C.

**8.** Process according to one of Claims 1 to 7, characterized in that the alcoholic medium or the alcohol fraction of the aqueous-alcoholic medium is formed by methanol, propanol, isopropanol, or by a mixture of these alcohols.

**9.** Process according to one of Claims 1 to 8, characterized in that the starting acid (II) is chosen from acrylic acid, methacrylic acid, maleic acid and fumaric acid.

**10.** Process according to Claim 7, leading to 3-mercaptopropionic acid, characterized in that the reaction is carried out at a temperature of 20 to 100°C.

**11.** Process according to one of Claims 1 to 10, characterized in that the acid (II) concentration, expressed in moles per litre of solvent (water or alcohol or water + alcohol), is between 0.5 and 4.